(19) Europäisches Patentamt
European Patent Office
Office européen des brevets

(11) **EP 3 981 324 B1**

(12) **EUROPEAN PATENT SPECIFICATION**

(45) Date of publication and mention
of the grant of the patent:
**08.01.2025 Bulletin 2025/02**

(21) Application number: **20818867.2**

(22) Date of filing: **08.06.2020**

(51) International Patent Classification (IPC):
*A61B 5/022* (2006.01)     *A61B 5/0245* (2006.01)
*A61B 5/00* (2006.01)     *A61B 5/024* (2006.01)
*A61B 5/11* (2006.01)

(52) Cooperative Patent Classification (CPC):
**A61B 5/1116; A61B 5/0077; A61B 5/022;
A61B 5/02416; A61B 5/6826**

(86) International application number:
**PCT/JP2020/022516**

(87) International publication number:
**WO 2020/246615 (10.12.2020 Gazette 2020/50)**

(54) **ASSESSMENT SYSTEM**

BEURTEILUNGSSYSTEM

SYSTÈME D'ÉVALUATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.06.2019 JP 2019107501**

(43) Date of publication of application:
**13.04.2022 Bulletin 2022/15**

(73) Proprietor: **DAIKIN INDUSTRIES, LTD.
Osaka-Shi, Osaka 530-0001 (JP)**

(72) Inventors:
• **GOTOU, Takashi**
**Osaka-shi, Osaka 530-8323 (JP)**
• **HASHIZUME, Hideki**
**Osaka-shi, Osaka 530-8323 (JP)**
• **HIRAYAMA, Takahiro**
**Osaka-shi, Osaka 530-8323 (JP)**
• **MATSUBARA, Akira**
**Osaka-shi, Osaka 530-8323 (JP)**
• **ARAI, Junichiro**
**Osaka-shi, Osaka 530-8323 (JP)**

(74) Representative: **Global IP Europe
Patentanwaltskanzlei
Pfarrstraße 14
80538 München (DE)**

(56) References cited:
EP-A1- 3 278 722     WO-A1-2016/163019
WO-A1-2018/127491     WO-A1-2018/163361
JP-A- 2015 054 223     JP-A- 2015 506 186
KR-A- 20190 050 725     US-A1- 2014 121 540
US-A1- 2014 358 017     US-A1- 2016 100 766
US-A1- 2016 228 011

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### TECHNICAL FIELD

The present invention relaates to a determination system.

### BACKGROUND ART

In order to detect orthostatic hypotension, it is necessary to measure a change in blood pressure at a time of a shift in posture, and blood pressure is conventionally measured by a contact type device (see Patent Literature 1 (JP 3121842U)). In addition, there is a continuous sphygmomanometer that is of a contact type and can measure a change in blood pressure chronologically.

US 2014/121540 A1, US 2016/228011 A1, US 2014/358017 A1, and US 2016/100766 A1 each disclose a facial change information acquirer that is configured to acquire facial change information indicating a chronological change in face data which is obtained from a face of the subject and related to a complexion of the subject, and that includes a color space processor configured to perform color separation processing of separating the facial change information into three color components of an red component, a green component, and a blue component.

WO 2018/127491 A1 discloses a sensor device configured to detect a patient's pulse by using a photoplethysmography sensor, and reduce noise components of the measured PPG signal based on motion signal indicating a motion of the patient and obtained by using a motion sensor.

### SUMMARY OF THE INVENTION

<Technical Problem>

Measuring a change in blood pressure at the time of a shift in posture requires a complicated measurement task. Thus, the measurement task is desired to be simple. In addition, because of the high price of the continuous sphygmomanometer, the measurement of the change in blood pressure is substituted by measurement of a heart rate change. As described above, it is desired to easily determine a physiological state of body such as a blood pressure.

<Solution to Problem>

A determination system according to claim 1 is disclosed.

The determination system may further include a first photographing unit that photographs a part of the body surface without contacting the subject. The body surface data is first photographed image data photographed by the first photographing unit.

The determination system may further include a second photographing unit that photographs the subject without contacting the subject. The motion detector detects the motion of the subject based on second photographed image data photographed by the second photographing unit.

The motion detector may be configured to detect the standing motion of the subject based on the face data.

### BRIEF DESCRIPTION OF THE DRAWINGS

FIG. 1 is a diagram of a determination system.
FIG. 2A is a diagram illustrating motions of a subject in processing of a control unit.
FIG. 2B is a flowchart illustrating a flow of the processing of the control unit.
FIG. 3A is a diagram illustrating an example of a measurement result.
FIG. 3B is a diagram illustrating an example of a measurement result.
FIG. 3C is a diagram illustrating an example of a measurement result.
FIG. 3D is a diagram illustrating an example of a measurement result.
FIG. 4A is a diagram illustrating another example of a measurement result.
FIG. 4B is a diagram illustrating another example of a measurement result.
FIG. 4C is a diagram illustrating another example of a measurement result.
FIG. 4D is a diagram illustrating another example of a measurement result.
FIG. 5A is a diagram illustrating another example of a measurement result.
FIG. 5B is a diagram illustrating another example of a measurement result.

FIG. 5C is a diagram illustrating another example of a measurement result.

FIG. 5D is a diagram illustrating another example of a measurement result.

FIG. 6 is a diagram of a determination system.

FIG. 7 is a schematic diagram illustrating how a fingertip surface of the subject is photographed by a body surface recording camera.

FIG. 8 is a flowchart illustrating a flow of processing of a control unit.

FIG. 9A is a diagram illustrating an example of a measurement result.

FIG. 9B is a diagram illustrating another example of a measurement result.

FIG. 9C is a diagram illustrating another example of a measurement result.

FIG. 9D is a diagram illustrating another example of a measurement result.

FIG. 9E is a diagram illustrating another example of a measurement result.

## DESCRIPTION OF EMBODIMENTS

<First embodiment>

(1) Overall configuration

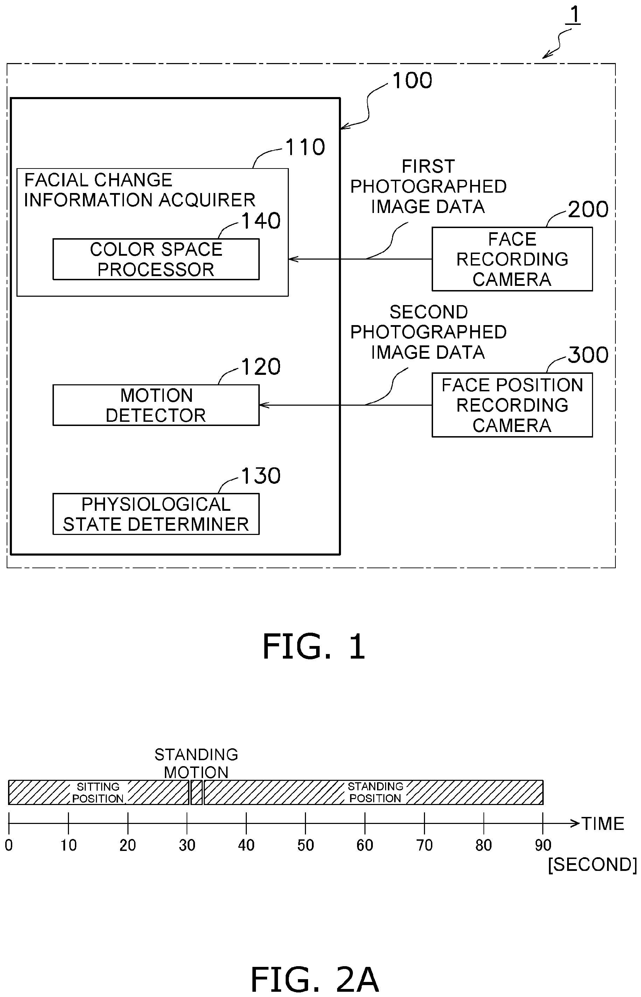

FIG. 1 illustrates a determination system 1 according to a first embodiment.

The determination system 1 determines a physiological state of mind or body by using image data (body surface data) obtained from a part of a body surface of a subject to be determined. The determination system 1 includes a control unit 100, a face recording camera 200, and a face position recording camera 300. The control unit 100 includes a facial change information acquirer 110, a motion detector 120, and a physiological state determiner 130.

Note that the control unit 100 can be implemented by a computer including a control calculator and a storage (neither illustrated). Examples of the control calculator include a processor such as a CPU or a GPU. The control calculator reads a control program stored in the storage and performs each processing in accordance with the control program. The control calculator can write a calculation result to the storage, and read information stored in the storage, in accordance with the control program.

First photographed image data is input from the face recording camera 200 to the facial change information acquirer 110. Second photographed image data is input from the face position recording camera 300 to the motion detector 120.

(2) Detailed configuration

(2-1) Facial change information acquirer

The facial change information acquirer 110 is a body surface change information acquirer that acquires facial change information indicating a chronological change in face data of the subject. Specifically, the facial change information acquirer 110 records chronologically the first photographed image data, which represent a photographed image of the subject photographed by the face recording camera 200, as face data that is body surface data related to a complexion of the subject, and acquires facial change information as body surface change information indicating a chronological change in the face data.

The facial change information acquirer 110 may use, as the face data, image data of a periphery of paranasal sinuses and/or a forehead of the subject included in the first photographed image data.

The facial change information acquirer 110 includes a color space processor 140. The color space processor 140 performs color separation processing of separating the facial change information into three color components, namely, an R component, a G component, and a B component.

The facial change information acquirer 110 acquires a component representing a characteristic of the physiological state and/or a conversion value, from color components included in the facial change information having been subjected to the color separation processing by the color space processor 140.

(2-2) Motion detector

The motion detector 120 detects a motion of the subject based on a height and a motion of a feature point of a predetermined region included in the second photographed image data. The motion of the subject refers to a height change of the head when the motion causes no change in a relative height between the heart and the head (for example, a motion from a sitting position to a standing position). The motion of the subject refers to a height change of the heart when the motion causes a change in the relative height between the heart and the head (for example, in a head-up tilt test of a head fixed type).

For example, when the subject stands up from a state of sitting on a chair, the feature point of the face of the subject included in the second photographed image data moves and rests still. As the height of the head of the subject changes, it is detected that the subject stands up.

(2-3) Physiological state determiner

The physiological state determiner 130 determines the physiological state of mind or body of the subject based on the face data acquired upon detecting the motion of the subject by the motion detector 120. Specifically, the physiological state determiner 130 determines the physiological state of mind or body of the subject based on the component representing the characteristic of the physiological state acquired by the facial change information acquirer 110 and/or the conversion value. The physiological state includes a decrease or an increase in a blood pressure, a pulse, a heart rate, and an autonomic nervous system activity of the subject.

(2-4) Face recording camera

The face recording camera 200 is a first photographing unit that photographs the subject without contacting (in non-contact with) the subject. The face recording camera 200 inputs the photographed first photographed image data to the facial change information acquirer 110.

(2-5) Face position recording camera

The face position recording camera 300 is a second photographing unit that photographs the subject without contacting (in non-contact with) the subject. The face position recording camera 300 inputs the photographed first photographed image data to the facial change information acquirer 110.

(3) Whole motion

FIG. 2A illustrates an example of a motion of the subject to be determined by the determination system 1. Hereinafter, determination processing performed by the determination system 1 will be described by exemplifying a case where the subject performs the motion illustrated in FIG. 2A. First, the subject sits on a chair and rests for 30 seconds (sitting position). Next, when a measurer instructs the subject to stand up, the subject stands up in about 3 seconds (standing motion). Next, the subject rests while standing for 60 seconds (standing position).

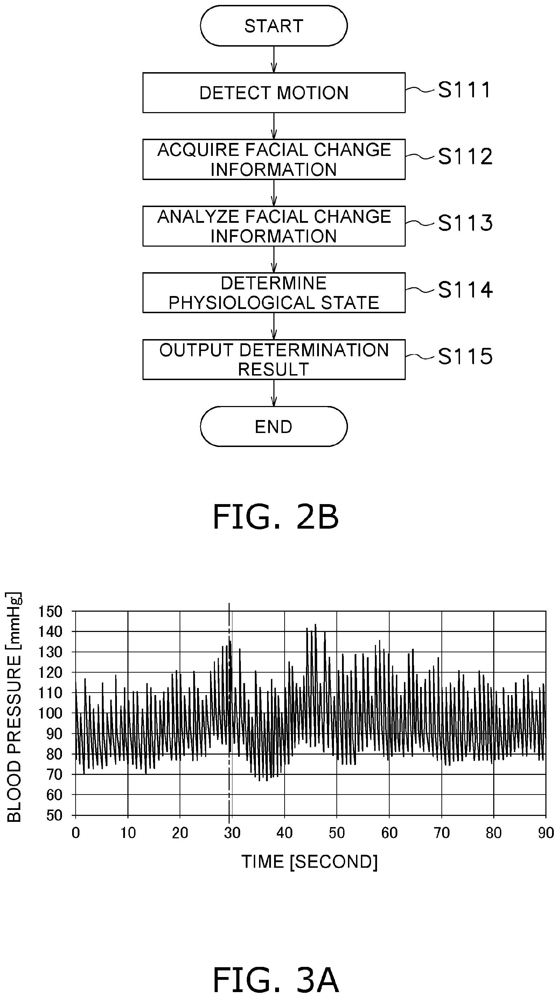

FIG. 2B illustrates a flow of processing of the control unit 100.

Upon start of processing by the control unit 100, the subject sits on a chair placed in front of the face recording camera 200 and the face position recording camera 300 (sitting position). When the control unit 100 starts the processing, the first photographed image data of the face of the subject is input from the face recording camera 200 to the facial change information acquirer 110. The second photographed image data of the subject is input from the face position recording camera 300 to the motion detector 120.

When the subject stands up from the state of sitting on the chair (standing motion), the motion detector 120 detects the motion of the subject based on the second photographed image data input from the face position recording camera 300 to the motion detector 120 (step S111).

Next, based on the first photographed image data input from the face recording camera 200 to the facial change information acquirer 110, the facial change information acquirer 110 acquires facial change information indicating a chronological change in the face data of the subject (step S112). At this time, the facial change information acquirer 110 uses, for example, image data of the periphery of the paranasal sinuses and/or the forehead of the subject included in the first photographed image data as the face data. The color space processor 140 of the facial change information acquirer 110 performs the color separation processing of separating the facial change information into three color components such as the R component (red component), the G component (green component), and the B component (blue component).

Next, the facial change information acquirer 110 analyzes the acquired facial change information (step S113). The facial change information acquirer 110 acquires the G component representing a characteristic of the physiological state and/or a conversion value such as an erythema index or a hemoglobin component, from the color components included in the facial change information having been subjected to the color separation processing by the color space processor 140.

The erythema index is an index representing a degree of "redness" of the skin using absorbance of the skin, and is, for example, an index obtained from RGB information by a calculation formula shown in the following Formula 1.

[Formula 1]

$$a *= 500 \left\{ \left( \frac{X}{X_n} \right)^{\frac{1}{3}} - \left( \frac{Y}{Y_n} \right)^{\frac{1}{3}} \right\}$$

$$X = 0.4124 * R + 0.3576 * G + 0.1805 * B$$

$$Y = 0.2126 * R + 0.7152 * G + 0.0722 * B$$

$$X_n = 98.071, Y_n = 100.0$$

The hemoglobin component is a value linked to an amount of hemoglobin estimated from the RGB information of the skin when it is assumed that the skin is of a two-layer model of a melanin layer and a hemoglobin layer.

Next, the physiological state determiner 130 determines the physiological state of the subject based on the face data acquired upon detecting the motion of the subject (step S114). The physiological state determiner 130 can determine the physiological state of mind or body of the subject such as a decrease or an increase in the blood pressure, the pulse, the heart rate, and the autonomic nervous system activity of the subject based on the component representing the characteristic of the physiological state acquired, from the face data, by the facial change information acquirer 110 and/or the conversion value before and after the motion of the subject.

Next, the control unit 100 outputs a determination result of the physiological state of mind or body of the subject by the physiological state determiner 130 to an output unit (not illustrated) (step S115), and ends the processing.

(4) Characteristics

(4-1)
The control unit 100 included in the determination system 1 according to the present embodiment includes the facial change information acquirer 110, the motion detector 120, and the physiological state determiner 130. The facial change information acquirer 110 acquires the facial change information indicating a chronological change in the face data of the subject. The motion detector 120 detects the motion of the subject. The physiological state determiner 130 determines the physiological state of mind or body of the subject based on the face data acquired upon detecting the motion of the subject.

The determination system 1 can easily determine the physiological state of mind or body of the subject. In addition, in this determination system, for example, it is possible to determine the physiological state of mind or body of the subject at low cost without using a device such as an expensive continuous sphygmomanometer.

(4-2)
In the determination system 1 according to the present embodiment, the facial change information acquirer 110 acquires, as face data, image data of the periphery of the paranasal sinuses and/or the forehead of the subject.

In the determination system 1, by acquiring data of a region of the face where a particularly large blood vessel exists, it is possible to easily determine the physiological state of mind or body of the subject.

(4-3)
In the determination system 1 according to the present embodiment, the face data is data related to the complexion of the subject.

In the determination system 1, since the complexion of the subject is known, it is possible to easily determine the physiological state of mind or body of the subject.

(4-4)
In the determination system 1 according to the present embodiment, the facial change information acquirer 110 includes the color space processor 140 that performs the color separation processing of separating the facial change information of the subject into three color components, namely, the R component, the G component, and the B component.

The determination system 1 can determine the physiological state of mind or body of the subject.

(4-5)
In the determination system 1 according to the present embodiment, the facial change information acquirer 110 acquires the G component and/or the conversion value of the erythema index, the hemoglobin component, or the like from the color component included in the facial change information subjected to the color separation processing.

The determination system 1 can easily determine the physiological state of mind or body of the subject by using the color component of the photographed image data in which the change in the complexion of the subject is easily detected.

(4-6)
In the determination system 1 according to the present embodiment, the motion of the subject is a motion including a height change of the head or the like of the subject.

In the determination system 1, the movement of the subject is easily detected, and then the physiological state of mind or body of the subject can be easily determined.

(4-7)
In the determination system 1 according to the present embodiment, the face recording camera 200 photographs in non-contact with the subject.

The determination system 1 can reduce a burden on the measurer and the subject.

(4-8)
In the determination system 1 according to the present embodiment, the physiological state includes a decrease or an increase in the blood pressure, the pulse, the heart rate, and the autonomic nervous system activity of the subject.

The determination system 1 can determine various physiological states.

(5) Modifications

(5-1) Modification 1A

In the determination system 1, the first photographed image data and the second photographed image data are obtained using the two cameras of the face recording camera 200 and the face position recording camera 300. However, the first photographed image data and the second photographed image data may be obtained using one camera.

(5-2) Modification 1B

In the present embodiment, the face recording camera 200 may be an infrared camera. This enables the first photographed image data to be obtained regardless of brightness of external environment. In this case, since the data from the infrared camera has one wavelength band, the processing proceeds to step S113 without performing step S112. Note that, there are a plurality of wavelength bands in a case where the infrared camera is a multi-wavelength camera. Thus, in step S112, the color space processor 140 performs decomposition processing of decomposing the facial change information into one or more predetermined wavelength band components included in an infrared wavelength band.

Then, in step S113, the facial change information acquirer 110 acquires a component representing the characteristic of the physiological state from the predetermined wavelength band components that are generated by the decomposition processing and included in the face change information.

Furthermore, in step S114, it is possible to determine the physiological state of mind or body of the subject, such as a decrease or an increase in the blood pressure, the pulse, the heart rate, and the autonomic nervous system activity of the subject, based on the component representing the characteristic of the physiological state acquired, from the face data, by the facial change information acquirer 110 before and after the motion of the subject.

(5-3) Modification 1C

The determination system 1 may further include a guide unit capable of keeping a constant distance between the subject and at least one of the face recording camera 200 and the face position recording camera 300 by contacting the subject and at least one of the face recording camera 200 and the face position recording camera 300. This allows photographing of the subject at a constant distance, and thus improves determination accuracy of the determination system 1.

<Second embodiment>

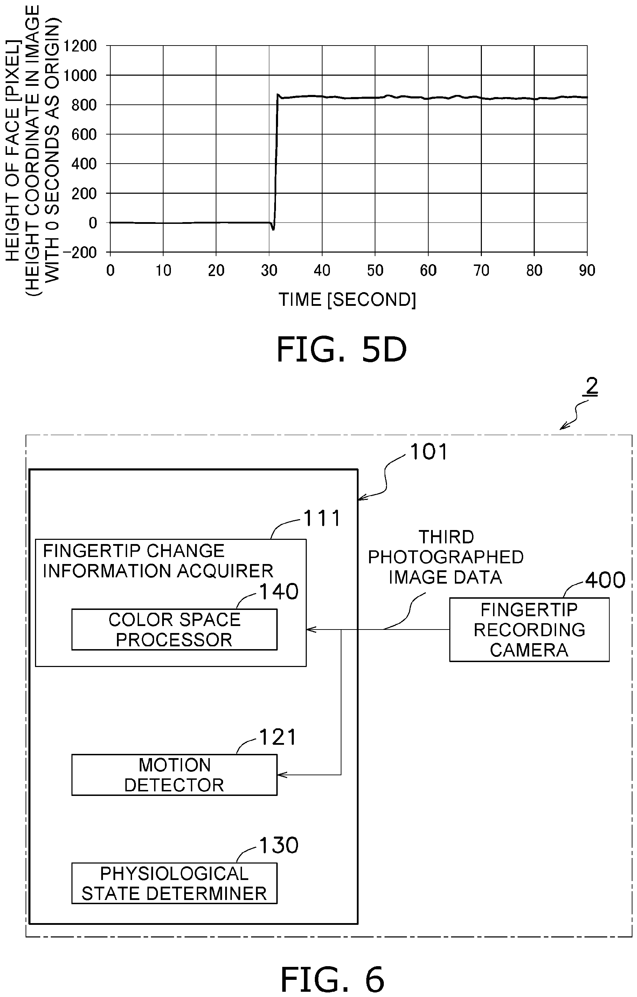

Next, a determination system 2 according to a second embodiment will be described focusing on a difference from the determination system 1. FIG. 6 is a diagram of the determination system 2. The difference between the determination system 1 and the determination system 2 is that the determination system 2 uses the image data of a fingertip surface of the subject to determine the physiological state of mind or body. Note that the same reference signs are given to the corresponding configurations in the respective embodiments, and the description thereof will be omitted.

(1) Overall configuration

The determination system 2 includes a control unit 101 and a fingertip recording camera 400. The control unit 101 includes a fingertip change information acquirer 111, a motion detector 121, and the physiological state determiner 130.

The fingertip recording camera 400 photographs an image of a surface of the fingertip of the subject and inputs the photographed image to the fingertip change information acquirer 111 and the motion detector 121 as third photographed image data.

(2) Detailed configuration

(2-1) Fingertip change information acquirer

The fingertip change information acquirer 111 is a body surface change information acquirer that acquires fingertip change information indicating a chronological change in fingertip data of the subject. Specifically, the fingertip change information acquirer 111 records chronologically the third photographed image data photographed by the fingertip recording camera 400 as fingertip data that is body surface data related to a color of the fingertip surface of the subject, and acquires fingertip change information that is fingertip change information indicating the chronological change of the fingertip data.

Similarly to the facial change information acquirer 110, the fingertip change information acquirer 111 includes the color space processor 140. The color space processor 140 performs color separation processing of separating the fingertip change information into three color components, namely, the R component, the G component, and the B component.

The fingertip change information acquirer 111 acquires a component representing the characteristic of the physiological state and/or a conversion value, from color components included in the fingertip change information having been subjected to the color separation processing by the color space processor 140.

(2-2) Motion detector

The motion detector 121 detects the motion of the subject based on the third photographed image data. Specifically, the motion detector 121 detects whether a predetermined pattern indicating the motion of the subject is included in the color component representing the characteristic of the physiological state and/or the conversion value acquired by the fingertip change information acquirer 111, and determines that there is a motion of the subject upon detection of the predetermined pattern.

Here, as the predetermined pattern indicating the motion of the subject, the motion detector 121 can use, for example, a pattern in which the R component decreases by a predetermined amount or more within a predetermined time. This pattern indicates occurrence of a phenomenon in which a blood flow of the fingertip physically decreases before the autonomic nerve acts due to the standing motion. Detection of this pattern from the R component allows the motion detector 121 to determine that there is a motion (standing motion) of the subject.

(2-3) Physiological state determiner

The physiological state determiner 130 determines the physiological state of mind or body of the subject based on the fingertip data acquired upon detecting the motion of the subject by the motion detector 121. Specifically, the physiological state determiner 130 determines the physiological state of mind or body of the subject based on the component representing the characteristic of the physiological state acquired by the fingertip change information acquirer 111 and/or the conversion value. The physiological state includes a decrease or an increase in a blood pressure, a pulse, a heart rate, and an autonomic nervous system activity of the subject.

(2-4) Fingertip recording camera

The fingertip recording camera 400 is a third photographing unit that photographs the fingertip surface of the subject. The fingertip recording camera 400 includes a sensor 401, lens 402, and an illumination 403.

The sensor 401 acquires an image of the fingertip surface via the lens 402. The fingertip recording camera 400 inputs the image acquired by the sensor 401 to the fingertip change information acquirer 111 and the motion detector 121. The illumination 403 irradiates the finger of the subject with light when an image is photographed. The lens 402 and the illumination 403 are disposed adjacent to each other so as to be touched by the fingertip simultaneously.

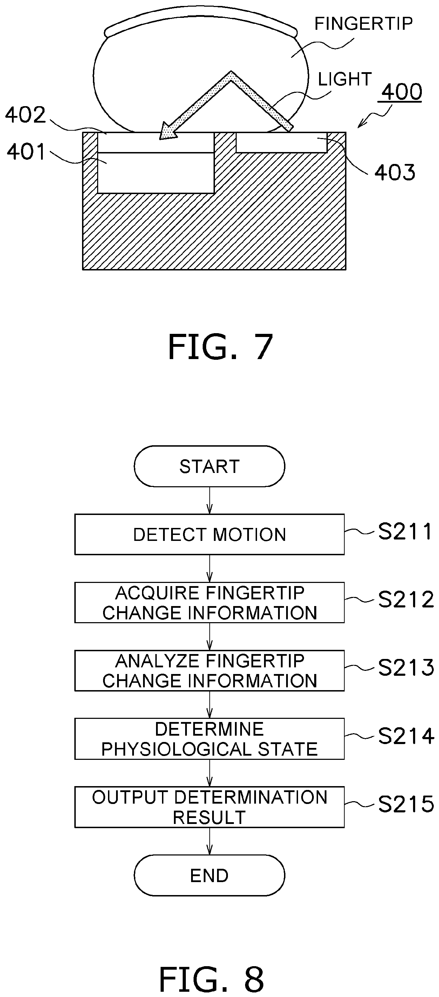

FIG. 7 is a schematic diagram illustrating how the fingertip surface of the subject is photographed by the fingertip recording camera 400. When the fingertip recording camera 400 acquires the third photographed image data, the subject touches the lens 402 and the illumination 403 with the fingertip surface as illustrated in FIG. 7. Accordingly, the light emitted from the illumination 403 is transmitted through the fingertip surface and then acquired by the sensor 401 via the lens 402.

A digital camera with a flash (illumination) attached to a smartphone may be used as the fingertip recording camera 400. In this case, the flash is used as the illumination 403.

(3) Whole motion

Processing of the control unit 101 will be described by exemplifying the motion of the subject illustrated in FIG. 2A.

FIG. 8 illustrates a flow of the processing of the control unit 101.

Upon start of processing by the control unit 101, the subject sits on a chair with the fingertip surface in contact with the fingertip recording camera 400 (sitting position). When the control unit 101 starts the processing, the third photographed image data is input from the fingertip recording camera 400 to the fingertip change information acquirer 111 and the motion detector 121.

When the subject stands up from the chair while the fingertip surface is still in contact with the fingertip recording camera 400 (standing motion), the motion detector 121 detects the motion of the subject based on the third photographed image data input from the fingertip recording camera 400 to the motion detector 121 (step S211).

Next, based on the third photographed image data input from the fingertip recording camera 400 to the fingertip change information acquirer 111, the fingertip change information acquirer 111 acquires the fingertip change information indicating the chronological change in the fingertip data of the subject (step S212). The color space processor 140 of the fingertip change information acquirer 111 performs the color separation processing of separating the fingertip change information into three color components such as the R component, the G component, and the B component.

Next, the fingertip change information acquirer 111 analyzes the acquired fingertip change information (step S213). The fingertip change information acquirer 111 acquires the R component representing a characteristic of the physiological state and/or a conversion value such as an erythema index or a hemoglobin component, from the color components included in the fingertip change information having been subjected to the color separation processing by the color space processor 140.

Next, the physiological state determiner 130 determines the physiological state of the subject based on the fingertip data acquired upon detecting the motion of the subject (step S214). Specifically, the physiological state determiner 130 can determine the physiological state of mind or body of the subject such as a decrease or increase in the blood pressure, the pulse, the heart rate, and the autonomic nervous system activity of the subject based on the component representing the characteristic of the physiological state acquired from the fingertip data by the fingertip change information acquirer 111 and/or the conversion value before and after the motion of the subject.

Next, the control unit 101 outputs a determination result of the physiological state of mind or body of the subject by the physiological state determiner 130 to an output unit (not illustrated) (step S215), and ends the processing.

(4) Characteristics

(4-1)

In the determination system 2, the body surface data is data obtained from the surface of the fingertip of the subject.

Therefore, the determination system 2 can obtain the determination result of the physiological state of mind or body by a simple operation.

(4-2)

In the determination system 2, the fingertip data can be acquired by the fingertip recording camera 400 being in contact with the fingertip.

Therefore, in the determination system 2, an influence of ambient light and the motion of the subject or the like can be suppressed, and the fingertip data can be obtained by a simple operation.

(4-3)

In the determination system 2, the motion detector 121 detects the motion of the subject based on the fingertip data.

The determination system 2, not requiring a camera for recording the position of the subject, simplifies a structure and suppresses a manufacturing cost.

(5) Modifications

(5-1) Modification 2A

In the determination system 2, in step S213, the color component acquired from the fingertip change information by the fingertip change information acquirer 111 is the R component. This is because the R component essentially transmits well through a living body and is easily detected by the sensor 401 without a sufficient light amount of the illumination 403.

Therefore, when the sensor 401 can detect without using the R component, such as when a sufficient light amount can be secured, the color component acquired from the fingertip change information by the fingertip change information acquirer 111 may be, for example, the G component or the B component other than the R component.

(5-2) Modification 2B

The fingertip recording camera 400 may be, for example, an infrared camera similarly to the face recording camera 200.

[Example 1]

The subject performed the motion illustrated in FIG. 2A, and the physiological state was determined by the determination system 1 according to the first embodiment. In addition, for evaluation of the determination system 1, the blood pressure and the heart rate were simultaneously measured by the continuous sphygmomanometer.

In this test, BP Monitor Ohmeda manufactured by Finapres Medical Systems was used as a continuous sphygmomanometer as a measuring instrument. As a recording apparatus, a digital oscilloscope DL 1640 manufactured by Yokogawa Electric Corporation was used. As the face recording camera 200, a camera A1H manufactured by Panasonic Corporation was used. As the face position recording camera 300, a camera WAT-01U2 manufactured by Watec Co., Ltd. was used. The continuous sphygmomanometer is a contact type.

The continuous sphygmomanometer is used in this test, but is not a constituent element of the whole system in FIG. 1.

FIGS. 3A to 3D illustrate an example of a measurement result of this test.

The subject is a healthy male person at age of 34.

FIG. 3A illustrates a result of measuring the blood pressure of the subject with the continuous sphygmomanometer. A vertical axis represents blood pressure (mmHg), and a horizontal axis represents time (seconds).

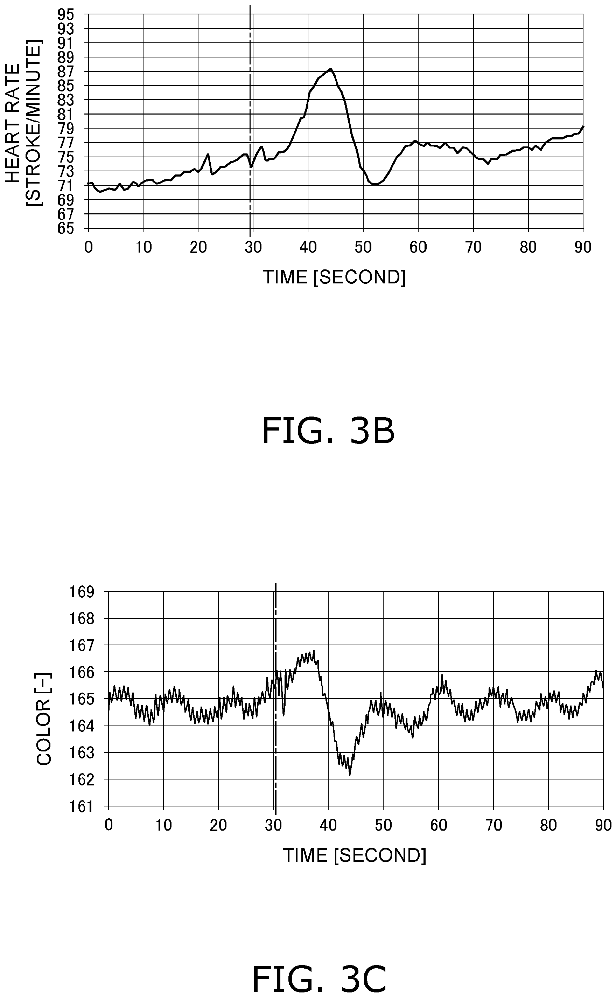

FIG. 3B illustrates a result of estimating the heart rate of the subject from the pulse rate. A vertical axis represents the heart rate (stroke/minute), and a horizontal axis represents time (second).

FIG. 3C illustrates a change in the complexion of the subject obtained by the control unit 100. A vertical axis represents the green component obtained by performing the color separation processing on the facial change information obtained from the first photographed image data input from the face recording camera 200. A vertical axis represents the green component included in the face data, and a horizontal axis represents time (second). The green component is an average of pixel values (gradation) of the green component in a plurality of pixels included in a predetermined range of the first photographed image data.

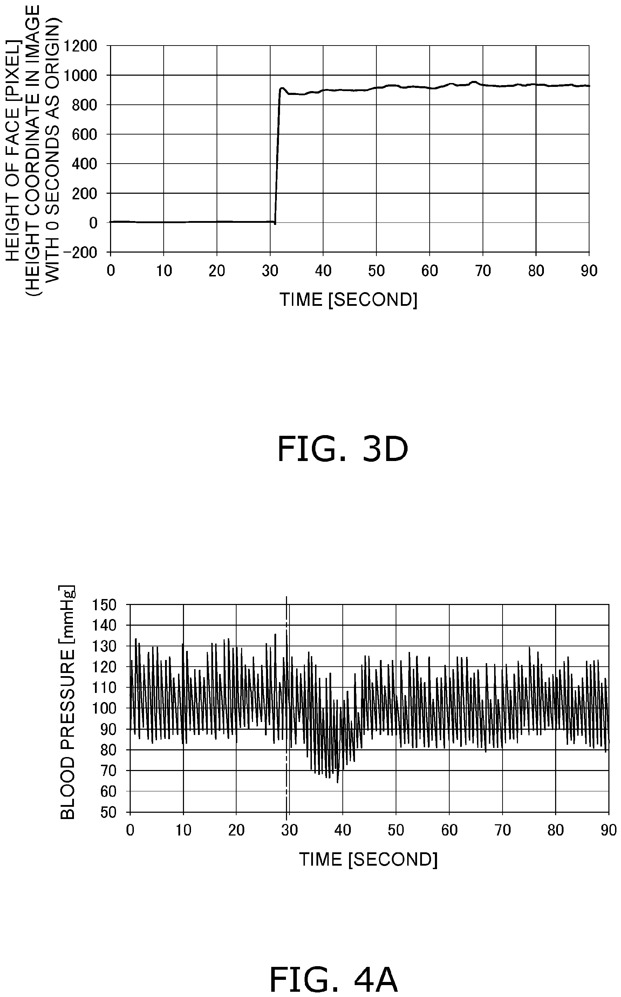

FIG. 3D illustrates a change in a height of the face of the subject obtained by the determination system 1. A vertical axis indicates a height coordinate in the second photographed image data with 0 seconds as an origin of the face in the second photographed image data photographed by the face position recording camera 300. A horizontal axis represents time (second).

As illustrated in FIG. 3D, sitting time is 0 seconds, and the height of the face is 0. Next, upon start of the measurement, since the subject is sitting on a chair and rests from 0 seconds to 30 seconds, the height of the face remains 0. When the subject performs the standing motion 30 seconds after the measurement is started, the height of the face becomes about 900 pixels. Thereafter, the subject is at rest while standing for 60 seconds, and thus the height of the face remains about 900 pixels from 30 seconds to 90 seconds.

In FIGS. 3A to 3C, vertical lines around 30 seconds after the start of the measurement indicate a timing at which the subject stands up from a state of sitting on the chair.

As illustrated in FIG. 3A, the subject stands up after 30 seconds elapse from the start of the measurement, and the blood pressure decreases from 30 seconds to 40 seconds. Thereafter, the blood pressure increases from 40 seconds to 50 seconds, and returns to a level of the blood pressure during the sitting position from 0 seconds to 30 seconds.

As illustrated in FIG. 3B, the subject stands up after 30 seconds elapse from the start of the measurement, and the heart rate increases from 30 seconds to 50 seconds. Thereafter, the heart rate decreases after 50 seconds elapse from the start of measurement, and returns to a level of the heart rate during the sitting position from 0 seconds to 30 seconds.

As shown in FIG. 3C, the subject stands up after 30 seconds elapse from the start of the measurement, and the green component of the complexion turns into increase from 30 seconds to 40 seconds. This indicates that when the subject stands up from a state of sitting on the chair, the blood pressure decreases and the complexion becomes blue. Thereafter, the green component of the complexion temporarily decreases and increases again from 40 seconds to 50 seconds. When 50 seconds or more elapse from the start of the measurement, the green component of the complexion returns to a level of the green component during the sitting position from 0 seconds to 30 seconds.

As described above, the blood pressure, the heart rate, and the complexion change at a timing at which the height of the face of the subject changes.

In this test, it has been confirmed that data on the complexion based on the face data obtained by the face recording camera 200 coincides with data obtained by measuring the blood pressure and the heart rate through contact measurement.

As shown in FIG. 3C, when a healthy person stands up from a state of sitting on the chair, the green component of the complexion increases and the complexion changes, but thereafter, the green component of the complexion immediately decreases and the complexion returns to an original state.

Here, in the detection of orthostatic hypotension, it is necessary to measure a change in blood pressure at a time of a shift in posture. The blood pressure of a healthy person temporarily decreases and returns to the original state in a short time due to a shift in posture. This physiological reaction prevents so-called "dizziness". In addition, the heart rate increases conversely and gradually returns to the original state. On the other hand, since the complexion changes substantially the same as the blood pressure, the determination system 1 according to the present embodiment is effective as, for example, non-contact and inexpensive detection of orthostatic hypotension. Specifically, in the determination system 1, the physiological state determiner 130 obtains a time from when the green component of the complexion increases due to the standing motion of the subject to when the green component of the complexion returns to the level of the green component during the sitting position based on the facial change information, and compares the obtained time with a predetermined reference time. When the time until the green component of the complexion returns to the level of the green component during the sitting position is longer than the reference time, the physiological state determiner 130 can determine that there is a risk of orthostatic hypotension in the subject.

[Example 2]

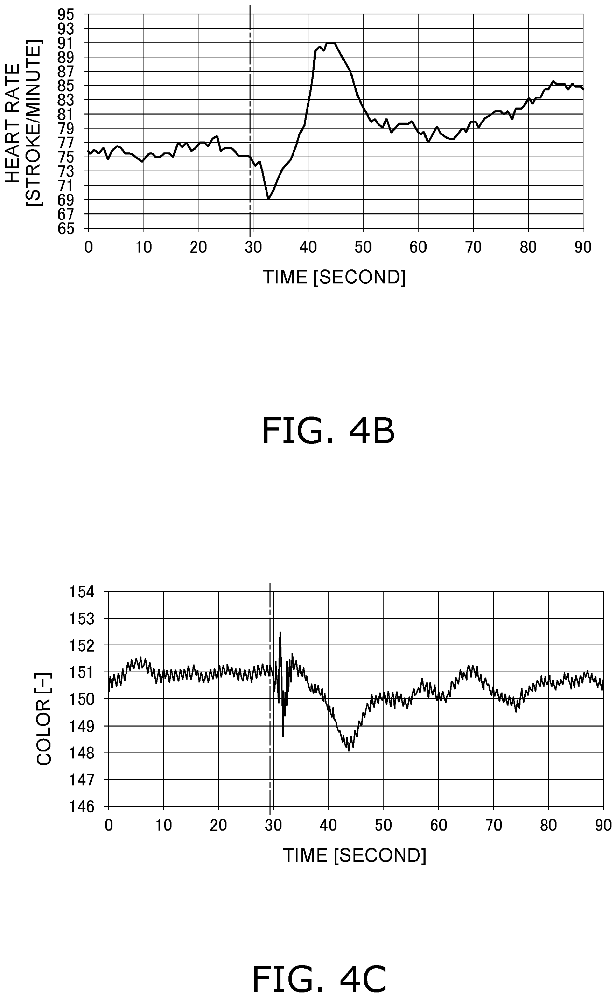

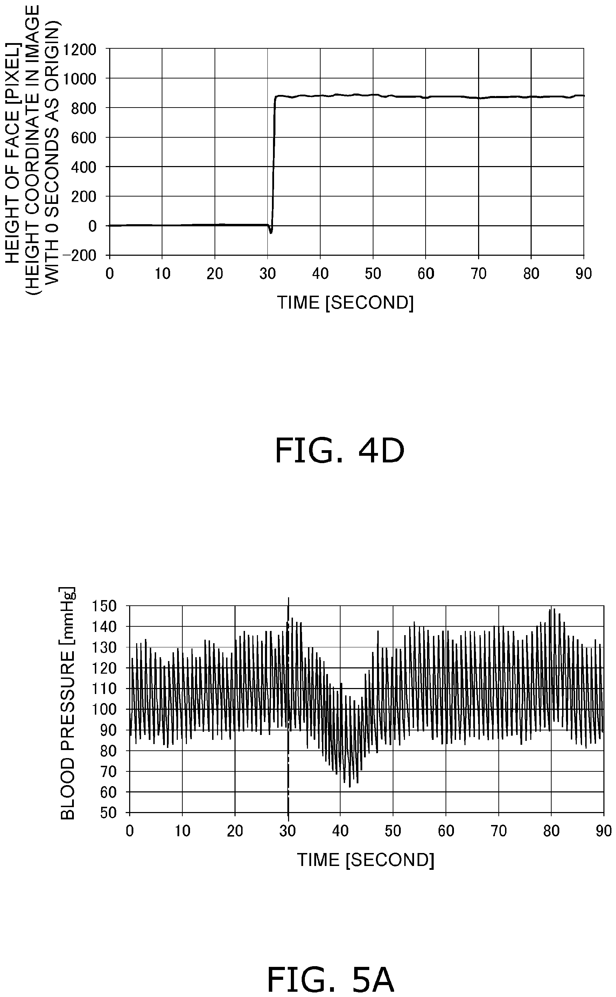

FIGS. 4A to 4D illustrate another example of the measurement result of this test.

The subject is a healthy male person at age of 33. The measurement results of the subjects in FIGS. 4A to 4D are almost the same as those in FIGS. 3A to 3D.

[Example 3]

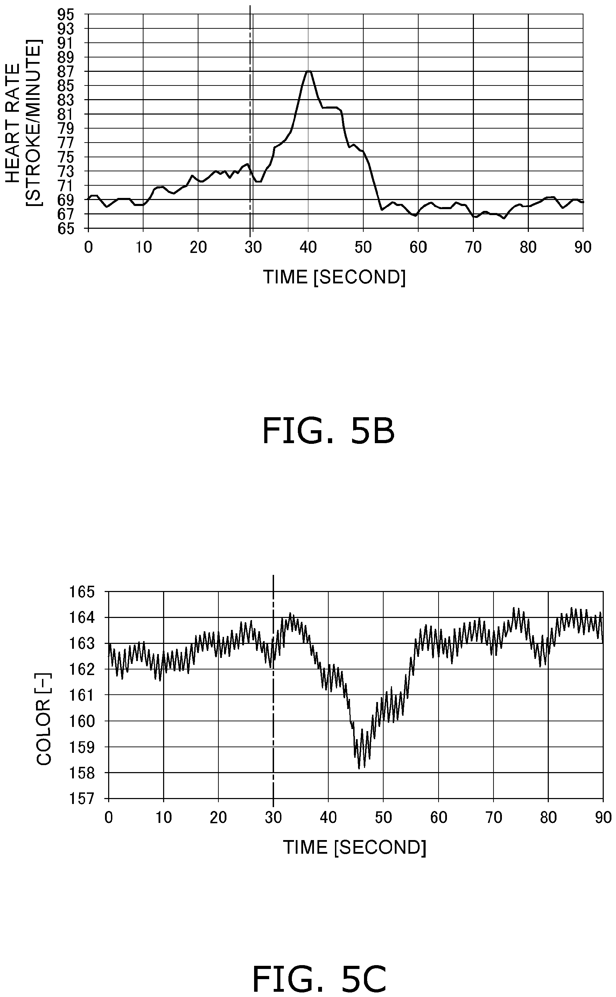

FIGS. 5A to 5D illustrate another example of the measurement result of this test.

The subject is a healthy male person at age of 52. The measurement results of the subjects in FIGS. 5A to 5D are almost the same as those in FIGS. 3A to 3D.

[Example 4]

The physiological state was determined by the determination system 2 according to the second embodiment. In addition, for evaluation of the determination system 2, the blood pressure was simultaneously measured by the continuous sphygmomanometer.

In the determination of the determination system 2, the subject performs a motion of sitting on a chair and resting for 60 seconds (sitting position), and when the measurer instructs to stand up, the subject performs five rounds of motions of standing up and then resting (standing position) for 60 seconds. In each interval of the rounds, the subject took a sufficient break.

In the test in Example 4, BP Monitor Ohmeda manufactured by Finapres Medical Systems was used as a continuous sphygmomanometer as a measuring instrument. As a recording apparatus, a digital oscilloscope DL 1640 manufactured by Yokogawa Electric Corporation was used. As the fingertip recording camera 400, a digital camera attached to iPhone (registered trademark) 6s manufactured by Apple Inc. was used. The continuous sphygmomanometer is a contact type.

At a time of photographing, a illumination next to the lens of the fingertip recording camera 400 was turned on. For photographing with the fingertip recording camera 400, a camera application installed as standard in the iPhone 6s was used. Photographing conditions were 720 p and 240 fps, and a codec was H.264 (lossless compression). The fingertip recording camera 400 was held at a height of the heart by the subject during the determination.

In the determination system 2 in Example 4, the erythema index was obtained from the formula shown in Formula 1 using the R component, the G component, and the B component.

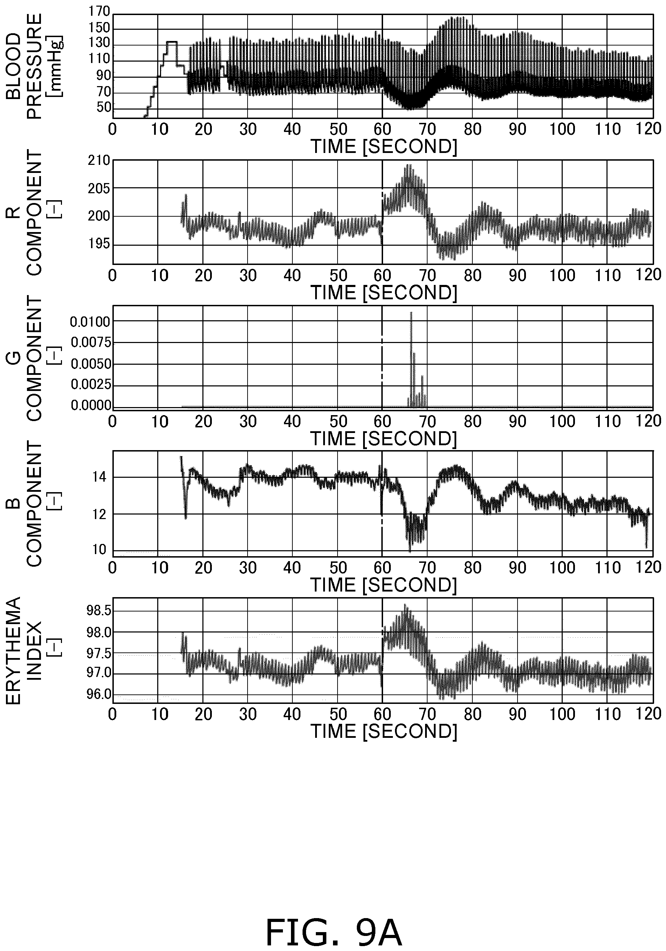

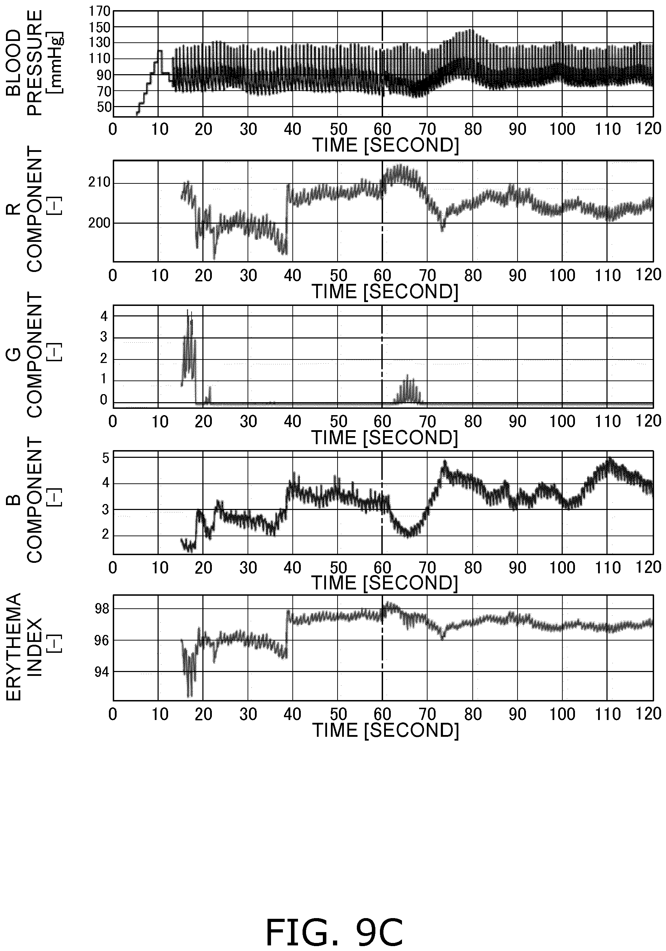

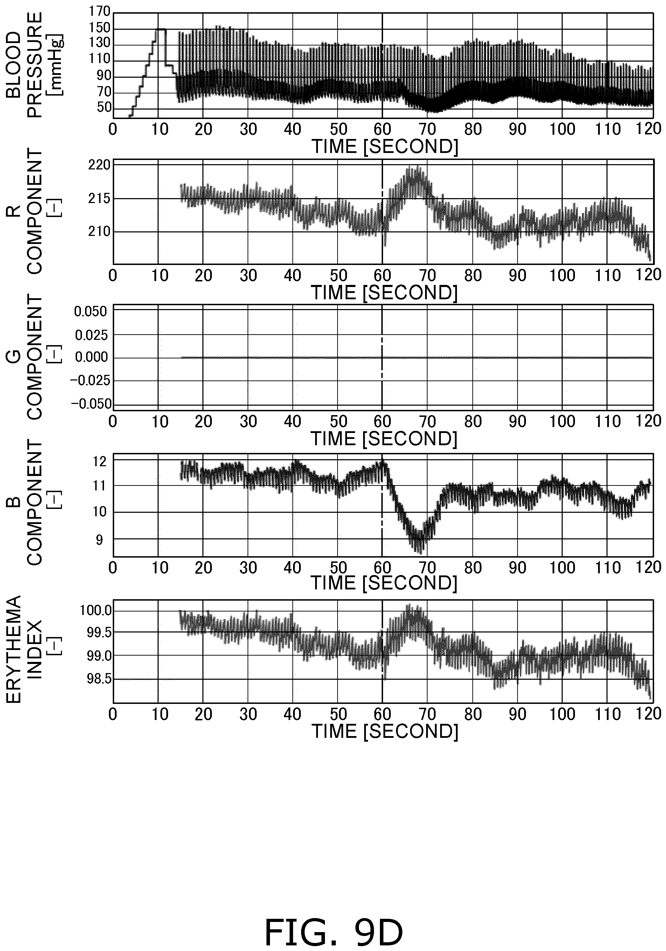

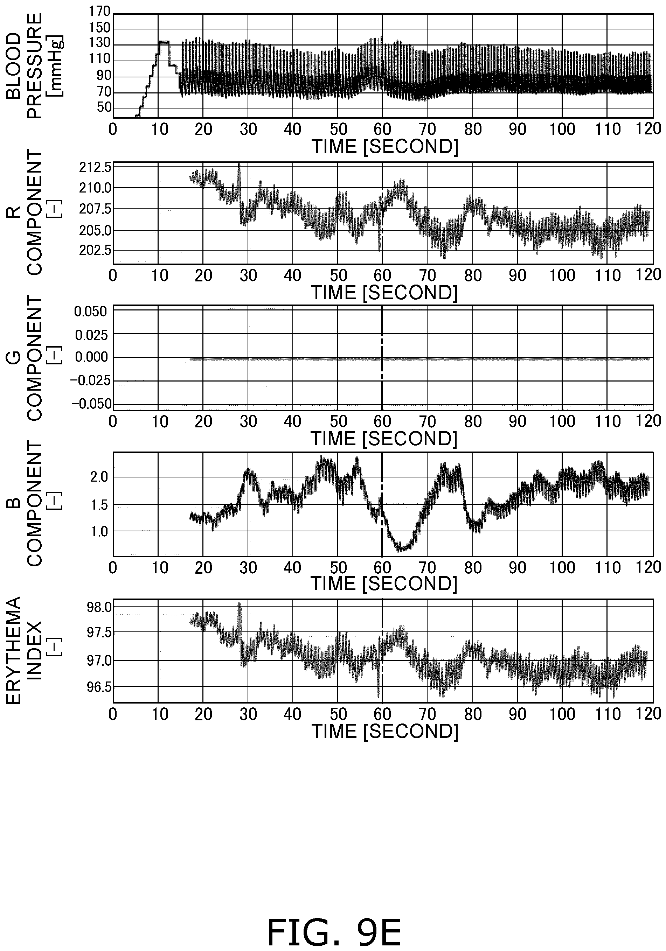

The continuous sphygmomanometer is used in this test, but is not a constituent element of the determination system 2. FIGS. 9A to 9E illustrate measurement results of the first to fifth tests of Example 4.

The subject is a healthy male person at age of 34.

FIGS. 9A to 9E illustrate the blood pressure, R component, G component, B component, and erythema index in order from the top.

The blood pressure is a result of measuring the blood pressure of the subject with the continuous sphygmomanometer, and a vertical axis represents the blood pressure (mmHg) and a horizontal axis represents time (seconds).

The R component, the G component, and the B component are values obtained by the fingertip change information acquirer 111 from the fingertip data obtained by the fingertip recording camera 400, and the vertical axis represents each color component and the horizontal axis represents time (seconds). Each color component is an average of pixel values (gradation) of each color component in a plurality of pixels included in a predetermined range of the third photographed image data.

The erythema index is a value obtained by the fingertip change information acquirer 111 from the R component, the G component, and the B component, the vertical axis represents the erythema index, and the horizontal axis represents time (seconds).

As illustrated in FIGS. 9A to 9E, when the subject stands up after 60 seconds elapse from the start of the measurement, the blood pressure decreases from 60 seconds to 70 seconds. Thereafter, the blood pressure increases between 70 seconds and 80 seconds, and returns to a level of the blood pressure during the sitting position from 0 seconds to 60 seconds.

As illustrated in FIGS. 9A to 9E, the subject stands up after 60 seconds elapse from the start of the measurement, and the R component turns into increase from 60 seconds to 70 seconds. This indicates that when the subject stands up from a state of sitting on the chair, the blood pressure decreases and the color of the fingertip becomes red. Thereafter, the R component temporarily decreases and increases again from 70 seconds to 80 seconds. When 90 seconds elapse from the start of the measurement, the R component returns to a level of the R component during the sitting position from 0 seconds to 60 seconds. The same tendency was observed for the erythema index obtained from the R component, the G component, and the B component.

It was confirmed by the test of Example 4 that data based on the fingertip data obtained by the fingertip recording camera 400 coincides with data obtained by measuring the blood pressure through contact measurement. It has been also confirmed that orthostatic hypotension can be determined by using the determination system 2 as well as using the determination system 1.

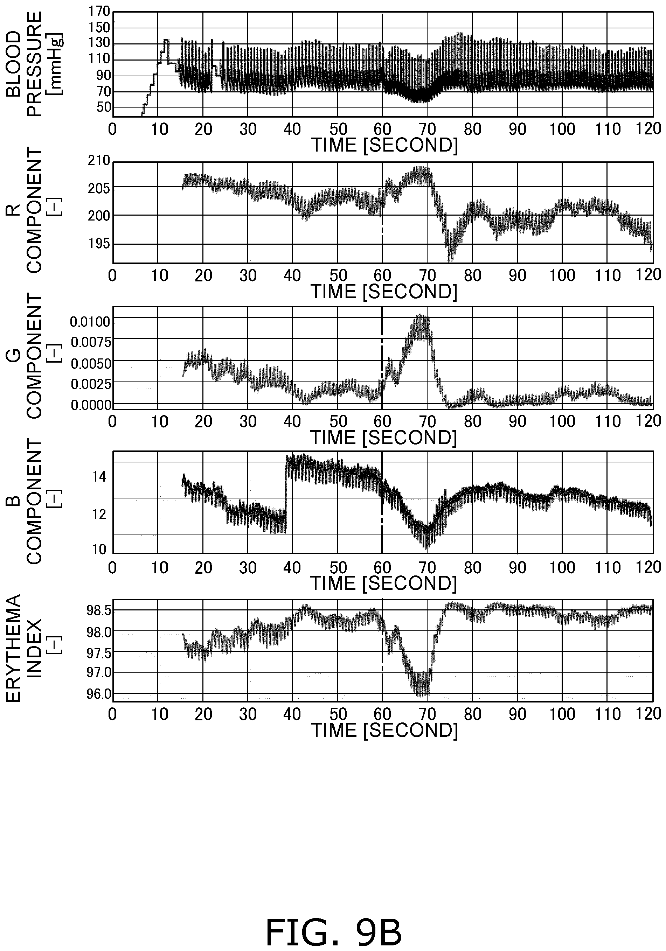

In the test of Example 4, a detection value of the G component was close to zero except for the second test illustrated in FIG. 9B. This is considered to be because the G component and the B component other than the R component are hardly transmitted through the living body, and a sufficient amount of light failed to reach the sensor 401.

The embodiments of the present disclosure have been described above. Various modifications to modes and details should be available without departing from the scope of the appended claims.

## REFERENCE SIGNS LIST

1, 2: determination system
100, 101: control unit
110: facial change information acquirer (body surface change information acquirer)
111: fingertip change information acquirer (body surface change information acquirer)
120: motion detector
130: physiological state determiner
140: color space processor
200: face recording camera (first photographing unit)
300: face position recording camera (second photographing unit)
400: fingertip recording camera

## CITATION LIST

### PATENT LITERATURE

Patent Literature 1: JP3121842U

## Claims

1. A determination system (1) comprising:

   a facial change information acquirer (110) that is configured to acquire facial change information indicating a chronological change in face data which is obtained from a face of the subject and related to a complexion of the subject, and that includes a color space processor (140) configured to perform color separation processing of separating the facial change information into three color components of an red component, a green component, and a blue component,
   a motion detector (120) configured to detect a standing up motion of the subject and **characterised by**
   a physiological state determiner (130) configured to

obtain a time from when the green component of the complexion increases due to the standing up motion of the subject to when the green component of the complexion returns to a level of the green component during a sitting position of the subject based on the facial change information,

compare the obtained time with a predetermined reference time, and

determine that there is a risk of orthostatic hypotension in the subject when the obtained time is longer than the predetermined reference time.

2. The determination system (1) according to claim 1, further comprising

a first photographing unit (200) configured to photograph the face without contacting the subject, wherein

the face data is first photographed image data photographed by the first photographing unit.

3. The determination system (1) according to claim 1, further comprising

a second photographing unit (300) configured to photograph the subject without contacting the subject, wherein

the motion detector (120) is configured to detect the standing motion of the subject based on second photographed image data photographed by the second photographing unit.

4. The determination system (1) according to claim 1 or 2, wherein
the motion detector (120) is configured to detect the standing motion of the subject based on the face data.

**Patentansprüche**

1. Bestimmungssystem (1), das aufweist:

einen Erfasser (110) für Gesichtsveränderungsinformationen, der konfiguriert ist, Gesichtsveränderungsinformationen zu erfassen, die eine chronologische Veränderung von Gesichtsdaten anzeigen, die von einem Gesicht der Person erhalten werden und mit einem Teint der Person in Zusammenhang stehen, und der einen Farbraumprozessor (140) enthält, der konfiguriert ist, eine Farbtrennverarbeitung des Trennens der Gesichtsveränderungsinformationen in drei Farbkomponenten einer Rotkomponente, einer Grünkomponente und einer Blaukomponente durchzuführen,

einen Bewegungsdetektor (120), der konfiguriert ist, eine Aufstehbewegung der Person zu erkennen, und **gekennzeichnet durch**

einen Bestimmer (130) des physiologischen Zustands, der konfiguriert ist, um:

eine Zeit von dem Zeitpunkt, zu dem die grüne Komponente des Teints aufgrund der Aufstehbewegung der Person ansteigt, bis zu dem Zeitpunkt, zu dem die grüne Komponente des Teints während einer sitzenden Position der Person auf einen Pegel der grünen Komponente zurückkehrt, basierend auf der Gesichtsänderungsinformation zu erhalten,

die erhaltene Zeit mit einer vorbestimmten Referenzzeit zu vergleichen und festzustellen, dass bei der Person das Risiko einer orthostatischen Hypotonie besteht, wenn die erhaltene Zeit länger als die vorbestimmte Referenzzeit ist.

2. Bestimmungssystem (1) nach Anspruch 1, das ferner aufweist:

eine erste Fotografiereinheit (200), die konfiguriert ist, das Gesicht zu fotografieren, ohne die Person zu berühren, wobei

die Gesichtsdaten erste fotografierte Bilddaten sind, die von der ersten Fotografiereinheit fotografiert wurden.

3. Bestimmungssystem (1) nach Anspruch 1, das ferner aufweist:

eine zweite Fotografiereinheit (300), die konfiguriert ist, die Person zu fotografieren, ohne die Person zu berühren, wobei

der Bewegungsdetektor (120) konfiguriert ist, die Aufstehbewegung der Person basierend auf zweiten fotografierten Bilddaten zu erkennen, die von der zweiten Fotografiereinheit fotografiert wurden.

4.  Bestimmungssystem (1) nach Anspruch 1 oder 2, wobei der Bewegungsdetektor (120) konfiguriert ist, die Aufstehbewegung der Person basierend auf den Gesichtsdaten zu erfassen.

**Revendications**

1.  Système de détermination (1), comprenant :

    une unité d'acquisition d'informations de modification faciale (110) configurée pour acquérir des informations de modification faciale indiquant un changement dans le temps de données faciales obtenues à partir du visage du sujet et relatives à la carnation du sujet, et comprenant un processeur d'espace colorimétrique (140) configuré pour exécuter un traitement de séparation des couleurs consistant à séparer les informations de modification faciale en trois composantes chromatiques, une composante rouge, une composante verte et une composante bleue,
    une unité de détection de mouvement (120) configurée pour détecter un mouvement de redressement du sujet,
    **caractérisé par**
    une unité de détermination d'état physiologique (130) configurée pour

    obtenir une durée entre le moment où la composante verte de la carnation croît en raison du mouvement de redressement du sujet et le moment où la composante verte de la carnation revient à un niveau de composante verte pendant une position assise du sujet, sur la base des informations de modification faciale, comparer la durée obtenue à une durée de référence prédéterminée, et
    déterminer qu'il existe un risque d'hypotension orthostatique chez le sujet si la durée obtenue est supérieure à la durée de référence prédéterminée.

2.  Système de détermination (1) selon la revendication 1, comprenant en outre une première unité photographique (200) configurée pour photographier le visage sans entrer en contact avec le sujet,
    où
    les données faciales sont des données d'image photographiées en premier par la première unité de photographie.

3.  Système de détermination (1) selon la revendication 1, comprenant en outre une deuxième unité photographique (300) configurée pour photographier le sujet sans entrer en contact avec lui,
    où
    le détecteur de mouvement (120) est configuré pour détecter le mouvement de redressement du sujet sur la base de deuxièmes données d'image photographiées par la deuxième unité de photographie.

4.  Système de détermination (1) selon la revendication 1 ou la revendication 2, où le détecteur de mouvement (120) est configuré pour détecter le mouvement de redressement du sujet sur la base des données faciales.

FIG. 1

FIG. 2A

START

DETECT MOTION — S111

ACQUIRE FACIAL CHANGE INFORMATION — S112

ANALYZE FACIAL CHANGE INFORMATION — S113

DETERMINE PHYSIOLOGICAL STATE — S114

OUTPUT DETERMINATION RESULT — S115

END

# FIG. 2B

# FIG. 3A

FIG. 3B

FIG. 3C

FIG. 3D

FIG. 4A

FIG. 4B

FIG. 4C

FIG. 4D

FIG. 5A

FIG. 5B

FIG. 5C

FIG. 5D

FIG. 6

FINGERTIP

LIGHT   400

402

401

403

# FIG. 7

START

DETECT MOTION — S211

ACQUIRE FINGERTIP
CHANGE INFORMATION — S212

ANALYZE FINGERTIP
CHANGE INFORMATION — S213

DETERMINE
PHYSIOLOGICAL STATE — S214

OUTPUT DETERMINATION
RESULT — S215

END

# FIG. 8

FIG. 9A

FIG. 9B

FIG. 9C

FIG. 9D

FIG. 9E

27

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3121842 U
- US 2014121540 A1
- US 2016228011 A1
- US 2014358017 A1
- US 2016100766 A1
- WO 2018127491 A1